# EUROPÄISCHE PATENTANMELDUNG

(11) **EP 2 998 284 A1**
(43) Veröffentlichungstag der Anmeldung: **23.03.2016**
(21) Anmeldenummer: 14185345.7
(22) Anmeldetag: 18.09.2014
(51) Int. Cl.: C07C 45/75, C07C 47/22

(54) **Optimiertes Verfahren zur Herstellung von Methacrolein**

(71) Anmelder: Evonik Röhm GmbH, 64293 Darmstadt (DE)
(72) Erfinder: Burghardt, Rudolf,Dr., 64287 Darmstadt (DE); Krill, Steffen, Dr., 664367 Mühltal (DE); Balduf, Torsten,Dr., 64319 Pfungstadt (DE); Kölbl, Gerhard, 64579 Gernsheim (DE); Köstner, Martin,Dr., 64295 Darmstadt (DE); Rundal, Eduard, 60486 Frankfurt (DE)

(57) **Zusammenfassung**

Die vorliegende Erfindung betrifft ein optimiertes Verfahren zur Herstellung von Methacrolein. Methacrolein wird in der chemischen Synthese insbesondere als Zwischenprodukt zur Herstellung von Methacrylsäure, Methylmethacrylat oder auch von Wirk-, Geruchs- oder Geschmacksstoffen verwendet. Insbesondere betrifft die vorliegende Erfindung die Optimierung der Prozessparameter, womit unter anderem eine Reduzierung des Gehalts an schädlichem dimeren Methacrolein im Endprodukt erzielt werden kann.

## Beschreibung

Die vorliegende Erfindung betrifft ein optimiertes Verfahren zur Herstellung von Methacrolein. Methacrolein wird in der chemischen Synthese insbesondere als Zwischenprodukt zur Herstellung von Methacrylsäure, Methylmethacrylat oder auch von Wirk-, Geruchs- oder Geschmacksstoffen verwendet. Insbesondere betrifft die vorliegende Erfindung die Optimierung der Prozessparameter, womit unter anderem eine Reduzierung des Gehalts an schädlichem dimeren Methacrolein im Endprodukt erzielt werden kann.

Es besteht ein großes Interesse an möglichst einfachen, wirtschaftlichen und umweltschonenden Herstellungsverfahren für Methacrolein.

### Stand der Technik

Bei der Herstellung von Methacrolein nach dem sogenannten C₂ -Verfahren wird aus Formalin und Propionaldehyd in Gegenwart eines sekundären Amins und einer Säure, zumeist einer organischen Säure, das Zielprodukt erhalten. Die Umsetzung erfolgt dabei über eine Mannich-Reaktion. Das so synthetisierte Methacrolein (MAL) kann dann in einem nachfolgenden Schritt durch Oxidation in der Gasphase zu Methacrylsäure oder durch oxidative Veresterung zu Methylmethacrylat umgesetzt werden. Ein solches Verfahren zur Herstellung von Methacrolein, ist unter anderem in den Druckschriften US 7,141,702, US 4,408,079, JP 3069420, JP 4173757, EP 0 317 909 und US 2,848,499 beschrieben.

Die zur Herstellung von Methacrolein geeigneten, auf einer Mannich-Reaktion basierenden Verfahren sind dem Fachmann im Allgemeinen bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2.

Für eine wirtschaftliche Nutzung dieses Verfahrens ist eine hohe Ausbeute und ein geringer spezifischer Energiebedarf zu erreichen. Nach der Lehre der EP 0 194 620 ist ein kleiner Gehalt an dimeren Methacrolein (DIMAL) im Produkt, bevorzugt kleiner 0,2 Gew%, bzw. ein Propionaldehydgehalt von kleiner 1 Gew% anzustreben, um eine nachhaltige Schädigung des Oxidationskatalysators der optional nachfolgenden heterogenen Gasphasenkatalyse zu vermeiden.

In DE 3213681 wird ein Verfahren zur Herstellung von MAL beschrieben, welches insbesondere dadurch charakterisiert ist, dass die Umsetzung bei einer Temperatur von größer 150 °C bei einer Reaktionszeit von höchstens 25 min in Gegenwart von sekundären Aminen und optional von Säuren durchgeführt wird. Bestenfalls wird dabei Propionaldehyd mit Formalin bei Temperaturen von 162 bis 205 °C sowie einer Verweilzeit von 6 Sekunden umgesetzt. Die Ausbeute beträgt im besten Fall 97,2 % und der DIMAL-Gehalt ist kleiner, jedoch nahezu 1 Gew%. Der Wassergehalt im Feed beträgt 40 Gew% und die auf den Wassergehalt bezogene Aminkonzentration beträgt 2,5 Gew%. Die deutlich geringe Ausbeute und der vergleichsweise hohe DIMAL-Gehalt zeigen, dass diese Fahrweise weniger vorteilhaft ist.

In einer weiteren Ausführungsform der DE 3213681 wird der Reaktor bei einer Eingangstemperatur von 161 °C betrieben und durch die stark exotherme Reaktion steigt die Temperatur auf bis zu 184 °C an. Die Verweilzeit beträgt ca. 6,9 sec. Der Wassergehalt im Feed zur Reaktion beträgt ca. 50 Gew%. Der auf das Wasser bezogene Amin-Gehalt beträgt 1,8 Gew%. So wird beispielsweise mit einer solchen Verfahrensvariante bei einer Ausbeute von 98,1 % ein DIMAL-Gehalt von 0,29 Gew% beobachtet. Nach der Lehre von EP 0 194 620 ist dieses MAL offenbar nicht ideal dazu geeignet, in einer heterogenen Gasphasenoxidation eingesetzt zu werden.

US 4,408,079 beschreibt ein Verfahren zur Herstellung von MAL, bei dem die Umsetzung von Propionaldehyd mit Formalin bei einem Molverhältnis von 0,9 bis 1,5 zu 1, einem pH-Wert zwischen 2,5 und 7 und Temperaturen von 0 °C bis 150 °C in Gegenwart von einem sekundären Amin, in einer Konzentration von 0,025 bis 0,75 bzw. von 0,05 bis 1,5 mol, und organischen Säuren in einer Konzentration von 0,05 bis 1,5 mol jeweils bezogen auf 1 mol Propionaldehyd durchgeführt wird. Im Vergleich zu der Lehre der DE 3213681 ist der gewählte Temperaturbereich somit deutlich tiefer. Dabei wird gemäß US 4,408,079 die Reaktion in einer Rührkesselkaskade von zwei bis drei Reaktoren kontinuierlich bei sehr langen Verweilzeiten der Reaktanden von 10 bis 90 min durchgeführt. Es werden mit dieser Ausführungsform des Verfahrens relativ niedrige Ausbeuten von 91 bis 96 % erzielt. Die Durchführung der Reaktion bei niedrigen Temperaturen zeigt somit erhebliche Nachteile. Neben einer deutlich verringerten Ausbeute sind die verwendeten Rührkesselreaktoren im Vergleich zu dem in DE 3213681 offenbarten Reaktor mit hohen Investitionskosten verbunden und aufwendig in der Wartung. Die DIMAL-Gehalte der Produkte werden nicht benannt. Beim Nacharbeiten der Beispiele wurden trotz der vergleichsweisen niedrigen Temperaturen hohe Gehalte von deutlich mehr als 1 Gew% von schädlichem dimeren Methacrolein gebildet. Ein solches Methacrolein dieser Qualität ist ohne weitere Aufarbeitung in keiner Folgestufe wirtschaftlich einsetzbar.

Damit besteht aus verschiedenen Gründen ein großes Interesse daran, ein Verfahren zur Herstellung von MAL zur Verfügung zu stellen, das geringere Mengen DIMAL als mit den Verfahren des Standes der Technik möglich, enthält. Dies spielt für eine weitere Verarbeitung des MAL in der Gasphasenoxidation zu Methacrylsäure insbesondere in Bezug auf die Wirkung als Katalysatorgift eine große Rolle. Insbesondere aber für die oxidative Veresterung des MAL zu MMA ist ein geringer DIMAL-Gehalt anzustreben, da DIMAL bzw. dessen Folgeprodukte auch in diesem Verfahren aufwendig vom Produkt abgetrennt werden müssen bzw. in erheblicher Art und Weise die Reaktion stören. Weiterhin führen DIMAL-Gehalte in der Aufarbeitung zu Färbung der Folgeprodukte.

### Aufgabe

In Anbetracht des Standes der Technik war es daher Aufgabe der vorliegenden Erfindung, ein Verfahren zur Herstellung von Methacrolein nach dem C₂-Verfahren zur Verfügung zu stellen, mit dem zum einen hohe Ausbeuten an Methacrolein erzeugt werden können und das Produkt zum anderen ohne weitere Aufarbeitungsverfahren in eine Gasphasenoxidation zu Methacrylsäure oder in eine oxidative Veresterung zu Methylmethacrylat (MMA) eingeleitet werden kann. Insbesondere bestand damit die Aufgabe, mit diesem Verfahren ein MAL herstellen zu können, welches ohne weitere Aufarbeitung einen DIMAL-Gehalt von kleiner 0,5 Gew%, bevorzugt kleiner 0,2 Gew% aufweist.

Weitere nicht explizit genannte Aufgaben ergeben sich aus dem Gesamtzusammenhang der nachfolgenden Beschreibung und der Ansprüche.

### Lösung

Gelöst werden diese Aufgaben mittels eines neuartigen Verfahrens zur kontinuierlichen Durchführung einer Mannich-Reaktion, mittels derer aus Formaldehyd und Propionaldehyd mit mindestens einer Säure und mindestens einer organischen Base als Katalysatoren Methacrolein hergestellt wird. Dabei wird erfindungsgemäß diese Reaktion in einem kontinuierlich betriebenen Rohrreaktor oder Plattenreaktor - im weiteren kurz als Reaktor bezeichnet - mit Temperaturgradienten durchgeführt. Die Eingangstemperatur des Zulaufs der Reaktanten und Katalysatoren in den Reaktor liegt dabei zwischen 100 und kleiner 150 °C, bevorzugt zwischen 110 und 140 °C und die Temperatur der Reaktionsmischung am Ablauf des Reaktors (Austrittstemperatur) liegt maximal bei 180°C, bevorzugt zwischen 150 und 180 °C, besonders bevorzugt zwischen 155 und 170 °C. Ganz besonders bevorzugt ist die im gesamten Reaktor vorliegende Maximaltemperatur 170 °C, insbesondere 165 °C. Weiterhin ist der Wassergehalt im Zulauf des Reaktors größer 45 Gew% und maximal 85 Gew% und die Menge an organischer Base im Zulauf des Reaktors beträgt bezogen auf 100 Mol% Propionaldehyd im Zulauf mehr als 5 Mol%. Die organische Base umfasst in diesem Zusammenhang neben der reinen Base den jeweiligen Basenanteil in den Salzen mit der Säure und in den basenhaltigen Zwischenprodukten der Mannich-Reaktion.

Der Innendruck des Reaktors ist darüber hinaus so eingestellt ist, dass dieser größer ist als der Siededruck der Reaktionsmischung. Anders ausgedrückt werden Druck und Temperatur derart eingestellt, dass die Umsetzung stets unterhalb des Siedepunktes des Reaktionsgemisches erfolgt, die Reaktion also in flüssiger Phase verläuft. Bevorzugt liegt in dem Reaktor ein Innendruck zwischen 10 und 50 bar, besonders bevorzugt zwischen 20 und 50 bar vor.

Unter Zulauf der Edukte in den Reaktor wird erfindungsgemäß der gesamte Zulauf, zusammengesetzt aus dem Frisch-Feed aus den für die Reaktion benötigten Edukten und dem optionalen Recyclingstrom verstanden. Die optionalen Recyclingströme werden z.B. aus der Aufarbeitung der Methacrolein-haltigen Mischung, welche aus dem Austritt des Reaktors entnommen ist, aus einer nachgeschalteten Destillationskolonne als wässrige Lösung aus dem Sumpf der Kolonne ganz oder teilweise zurück in den Reaktor geführt werden. Dies ist insbesondere bei den weiter unten folgenden Angaben zur Zusammensetzung des Zulaufs zu beachten. Ein kontinuierlich betriebener Reaktor wird häufig, im Falle der beschriebenen Mannich-Reaktion sogar bevorzugt unter zumindest teilweiser Zurückführung von Recyclingströmen betrieben. Dadurch bildet sich bei konstantem Betrieb eine stationäre Phase im Reaktor, die von der Zusammensetzung des Frisch-Feeds, insbesondere in Bezug auf die Katalysatorkomponenten, derart abweichen kann, dass im Reaktor eine höhere Katalysatorkonzentration vorliegt, als die im Frisch-Feed eingeleitete. Der erwähnte Frisch-Feed setzt sich in der Regel zusammen aus einem Propionaldehyd-Strom, einem Formalin-Strom, einem Strom der organischen Base und dem Säure- Strom. Dabei kann es bezüglich der Zuleitung dieser Ströme in den Reaktor unterschiedliche Ausführungsformen geben. Zum einen ist es möglich, dass alle oder ein Teil der Komponenten bereits als Mischung vorliegen und zusammen in den Reaktor geleitet werden. Alternativ ist es auch möglich, dass alle oder der nicht als Mischung vorliegende Anteil der Edukte in Einzelströmen direkt in den Reaktor geleitet werden. Ferner ist auch eine Ausführung möglich, bei der alle oder einige der Edukte in einer dem Reaktor vorgeschalteten Mischkammer mit einander vermischt werden oder in eine gemeinsame Leitung zusammengeführt werden, bevor sie in den Reaktor geleitet werden. Dabei ist jedoch darauf zu achten, dass, wenn alle Komponenten miteinander bei der Eingangstemperatur vermischt werden, die Verweilzeit außerhalb des Reaktors besonders kurz zu halten ist, da hier die Reaktion gestartet wird und somit der eigentlichen Verweilzeit zuzurechnen ist.

In besagter optionaler Mischkammer kann die Mischung der Monomere mittels eines statischen oder dynamischen Mischers unterstützt werden. Auch kann die Mischung mittels einer dazu ausgelegten Eindüsung in die Mischkammer erfolgen. Weiterhin ist es möglich, die Mischung mittels einer solchen Eindüsung direkt am Eingang des Reaktors zu bewirken.

Die Edukte werden in der Regel rein oder als Lösung, insbesondere als wässrige Lösung eingesetzt. Insbesondere das Formaldehyd wird in der Regel als Formalin, also als wässrige Lösung, mit einer Konzentration zwischen 35 und 60 Gew% eingesetzt.

Die Verweilzeit der Reaktionsmischung in dem Reaktor sollte trotz der gegenüber dem Stand der Technik geringeren Eingangstemperatur eher kurz gehalten werden. Insbesondere liegt die Verweilzeit in dem Reaktor zwischen 1 und 30 s, bevorzugt zwischen 5 und 15 s und ganz besonders bevorzugt zwischen 7 und 12 s. Zu kurze Verweilzeiten gehen auf Kosten der Gesamtausbeute oder aber bedingen eine zu hohe Eingangstemperatur in den Reaktor bzw. zu hohe Maximaltemperatur im Reaktor, um einen vollständigen Umsatz der Reaktanten zu gewährleisten. Überraschend wurde darüber hinaus beobachtet, dass mit kürzeren Verweilzeiten im Reaktor auch die Bildung des dimeren MAL unterdrückt wird. Damit sind eher kürzere Verweilzeiten in den beschriebenen Grenzen bevorzugt für die Reaktionsführung.

Erfindungsgemäß ist es insbesondere eine überraschend effektive Änderung gegenüber dem Stand der Technik, dass die Ausgangsstoffe mit einer deutlich geringeren Temperatur in den Reaktor gefahren werden, während die Verweilzeit innerhalb des Reaktors überraschend nicht relevant verlängert werden muss. Besonders überraschend wurde dabei festgestellt, dass eine solche Reaktionsführung zur Bildung deutlich geringerer Mengen dimeren Methacroleins führt.

Bezüglich der Zusammensetzung der Reaktionsmischung sind insbesondere Zusammensetzungen im Zulauf des Reaktors mit einem Verhältnis von Propionaldehyd zu Formaldehyd zwischen 0,75 zu 1 mol und 1 zu 1,2 mol, bevorzugt zwischen 1 zu 0,98 mol und 1 zu 1,02 mol.

Genauso bevorzugt sind Zusammensetzung des Zulaufs, in denen bezogen auf ein mol Propionaldehyd zwischen 0,05 und 0,15 mol, bevorzugt zwischen 0,06 und 0,1 mol und besonders bevorzugt zwischen 0,06 und 0,08 mol Base enthalten sind. Bevorzugt stammen mindestens 20 % der Gesamtmenge organischer Base des Zulaufs aus dem Frisch-Feed und entsprechend bevorzugt der Rest aus dem Recyclingstrom. Weiterhin bevorzugt ist ein Zulauf, in dem bezogen auf ein mol organische Base zwischen 0,8 und 1,5, bevorzugt zwischen 0,9 und 1,3 und besonders bevorzugt zwischen 1 und 1,2 mol Säure enthalten sind. Das Verhältnis der Äquivalente Amin zu Säure und deren konkrete Auswahl wird vorzugsweise so gewählt, dass im Reaktionsgemisch vor der Reaktion, gemessen bei 20 °C und Normaldruck, ein pH-Wert von 3,0 bis 7,0, bevorzugt von 3,5 bis 6,5 resultiert.

Bei den Säuren handelt es sich in der Regel um anorganische Säuren oder organische Mono-, Di- bzw. Polycarbonsäuren, bevorzugt Monocarbonsäuren, insbesondere aliphatische Monocarbonsäuren. Besonders bevorzugt wird zur Umsetzung von Propanal und Formaldehyd mindestens eine organische Säure, besonders bevorzugt Ameisensäure, Essigsäure und/oder Propionsäure, ganz besonders bevorzugt Essigsäure eingesetzt.

Bei den organischen Basen handelt es sich vorzugsweise um Amine, besonders bevorzugt um sekundäre Amine. Als Amine kommen beispielsweise in Betracht: Dimethylamin, Diethylamin, Methylethylamin, Methylpropylamin, Dipropylamin, Dibutylamin, Di-iso-propylamin, Di-iso-butylamin, Methyl-iso-propylamin, Methyl-iso-butylamin, Methyl- sek.-butylamin, Methyl-(2-methylpentyl)-amin, Methyl-(2-ethylhexyl)-amin, Pyrrolidin, Piperidin, Morpholin, N-Methylpiperazin, N-Hydroxyethylpiperazin, Piperazin, Hexamethylenimin, Diethanolamin, Methylethanolamin, Methylcyclohexylamin, Methylcyclopentylamin, Dicyclohexylamin oder entsprechende Gemische. Eine besonders bevorzugte organische Base ist Dimethylamin.

Der Wassergehalt des Zulaufs aus Frisch-Feed und Recyclingstrom liegt wie beschrieben erfindungsgemäß zwischen 45 und 85 Gew%. Bevorzugt liegt der Wassergehalt des Zulaufs zwischen 50 und 70 Gew%. Als besonders vorteilhaft haben sich Wassergehalte im Zulauf zwischen 55 und 65 Gew% erwiesen.

Der erfindungsgemäß verwendete Rohrreaktor weist bevorzugt einen Innendurchmesser eines Einzelrohrs zwischen 4 und 20 mm, besonders bevorzugt zwischen 8 und 15 mm auf. Insbesondere werden mehrere Rohrreaktoren in Form eines Rohrbündelreaktors eingesetzt. Alternativ zum Rohrbündelreaktor oder einzelnen Rohrreaktoren kann das erfinderische Verfahren weniger bevorzugt auch in einem Plattenreaktor mit Wärmetauscher, insbesondere mit einem Plattenabstand zwischen 0,5 und 10 mm, bevorzugt 1 bis 5 mm, durchgeführt werden. Dabei kann es sich bei dem Plattenreaktor auch um einen vollverschweißten Plattenwärmetauscher mit ausreichender Druckfestigkeit handeln.

Als besonders vorteilhaft hat es sich dabei erwiesen, wenn die Rohrreaktoren mit einer Leerrohrgeschwindigkeit zwischen 0,3 und 2,0 m/s, bevorzugt von 0,8 bis 1,2 m/s, ganz besonders bevorzugt zwischen 0,85 und 0,90 m/s durchströmt werden. Ganz besonders bevorzugt weisen die Rohrreaktoren zusätzlich optional statische Mischer auf.

Ganz besonders vorteilhaft ist es, wenn der Reaktor vom Zulauf ausgehend mindestens eine adiabatische Zone, eine darauf folgende Kühlzone und eine zweite adiabatische Zone am Austritt aufweist. Die Kühlung, insbesondere in der mittleren Zone, erfolgt dabei ganz oder teilweise mit Hilfe eines Kühlkreislaufs. Dieser Kühlkreislauf kann verschiedentlich ausgestaltet sein und in der spezifischen Konstruktion für den Fachmann einfach planbar sein. Zum einen ist es möglich ein vorgeheiztes Kondensat in den Kühlmantel ein- und erzeugten Dampf wieder auszuleiten. Auch ist es möglich eine Kühlflüssigkeit, insbesondere Wasser oder ein bei den Betriebstemperaturen stabiles ÖL derart unter Druck im Kreis zu führen, dass die Kühlflüssigkeit auch beim Erhitzen flüssig bleibt. Die Innentemperatur und dessen Gradient werden insbesondere eingestellt durch eine Kombination des Wärmedurchgangskoeffizienten, der gewählten Kühltemperatur, sowie die Kühlungsart und durch die Wahl der Betriebsart des Reaktors in Bezug auf einen Kühlmantel im Gleichstrom oder im Gegenstrom. In der Regel wird die Kühlflüssigkeit mit einer Temperatur zwischen 100 und 140 °C in den Kühlmantel eingeleitet.

Gleichsam bevorzugt ist eine Methacroleinkonzentration der Reaktionsmischung am Ablauf zwischen 20 und 50 Gew%.

Bei der Herstellung von Methacrolein aus Propanal und Formaldehyd wird bevorzugt das im Reaktor umgesetzte Reaktionsgemisch aus dem Austrag in eine Kolonne geleitet und dort mit dem aus dem mit aus dem eingeleiteten Wasser gebildeten Wasserdampf gestrippt. Das Produkt verlässt zusammen mit Wasser die Kolonne am Kopf. Das Gemisch wird kondensiert und über ein Phasentrenngefäß in eine obere und eine untere Phase getrennt. Die obere Phase enthält das Methacrolein. Die untere Phase besteht hauptsächlich aus Wasser. Vorzugsweise kann dieses Wasser zur Entfernung des noch darin gelösten Produktes zumindest teilweise, bevorzugt ganz, wieder in die Kolonne zurückgeführt werden. Ein Teil der wässrigen Phase im Sumpf wird in einem solchen Fall kontinuierlich oder batchweise abgeführt und einer Aufarbeitung bzw. einer Entsorgung zugeleitet. Bei diesen kann es sich um eine Membrantrennstufe, eine weitere Destillation, eine - abhängig von den enthaltenen Bestandteilen - biologische Entsorgung oder einen Thermal Oxidizer handeln.

Als besonders vorteilhaft hat es sich erwiesen, wenn mindestens 20 Gew%, bevorzugt mindestens 50 Gew% und besonders bevorzugt mindestens 60 Gew% der wässrigen Phase aus dem Sumpf in den Eintritt des Rohrreaktors recycliert werden. Dieser prozentuale Anteil wird im Weiteren als Recyclierungsrate bezeichnet. Es hat sich überraschend gezeigt, dass durch diese Maßnahme und die damit verbundene zusätzliche Verdünnung der Reaktionslösung bei gleichzeitig nahezu gleichbleibender oder steigender Katalysatorkonzentration mit dem erfindungsgemäßen Verfahren noch weniger dimeres Methacrolein gebildet wird und die Selektivität der Reaktion nochmal höher ist.

Zusätzlich kann der Reaktor weitere Zonen aufweisen. So ist insbesondere eine optionale Wärmeübertragungszone zu nennen, in der die Reaktionsmischung kälter ist als die Kühlflüssigkeit des Reaktormantels. Eine solche Zone kann sich bei einem relativ kühlen Zulauf mit einer Temperatur von 100 °C oder nur wenig wärmer z.B. direkt hinter der ersten adiabaten Zone ergeben. Alternativ kann die Wärmeübertragungszone auch vor der ersten adiabatischen Zone als Vorwärmzone angeordnet sein.

Die genau einzustellende Recyclierungsrate ergibt sich aus dem gewünschten Wassergehalt im Reaktor und den Wassermengen, die über die Edukte und Katalysatorkomponenten in den Reaktor eingebracht werden. Dies betrifft insbesondere das Formalin. Es ist jedoch auch möglich die organische Base und/oder die Säure als wässrige Lösungen einzuspeisen. Weiterhin kann auch das Propionaldehyd z.B. bis zu 5 Gew% Wasser aufweisen.

Das im Reaktor bei der Herstellung von Methacrolein aus Propanal und Formaldehyd vorliegende Wasser setzt sich aus dem Wasser, welches als Katalysatorlösung zugegeben wurde, dem bei der Reaktion gebildeten Reaktionswasser und optional dem aus der Formaldehydlösung enthaltenen Wasser zusammen. Weitere, jedoch in geringerem Umfang zu berücksichtigende Wasserquellen sind Bestandteile der technischen Edukte wie Propanal und Wasser, das in diversen Nebenreaktionen der Katalysatorkomponten mit Edukten, Nebenprodukten und Reaktionsprodukten gebildet wird, sowie Reaktionswasser aus allen diesen Komponenten, die unter den Reaktionsbedingungen entstehen

Nebenprodukte der Reaktion können verschiedentlich entstehen. Hier sind zunächst als Beispiel vor allem Nebenprodukte der Katalysatorkomponenten, wie beispielsweise höher alkylierte Amine, wie insbesondere Trimethylamin bei Verwendung von Dimethylamin als originärem Katalysatoramin, aufzuführen. Auch kleine Mengen Edukte können in dem Austrag enthalten sein. Beispiele hierfür sind Methacrolein, Formaldehyd, Paraformaldehyd und Propanal. Als Nebenprodukte der Reaktion, die gleichsam in dem aminhaltigen Reaktionswasser enthalten sind, wären beispielsweise Dimere, Oligomere oder Polymere des Methacrolein zu nennen. Weiterhin können je nach Prozessführung auch weitere Hilfsstoffe, wie organische Lösungsmittel, wie z.B. Methanol, Ameisensäure, Propanol, Dioxan, Tetrahydrofuran oder Methoxyethanol enthalten sein - sowie weitere in der Reaktionsmatrize enthaltende oder entstehende Stoffe.

Ein besonders überraschender Vorteil der vorliegenden Erfindung ist, dass durch die erfindungsgemäße Reaktionsführung insbesondere schwer abtrennbare und/oder direkt die Ausbeute beeinträchtigende Nebenprodukte in deutlich geringerem Maße gebildet werden, als aus Verfahren des Standes der Technik bekannt ist. Damit kann das Verfahren insgesamt mit einer hohen Ausbeute durchgeführt werden, ohne dass besonders aufwendige Reinigungsschritte notwendig sind.

Insbesondere wurde dabei überraschend gefunden, dass durch die erfindungsgemäße Reaktionsführung der Anteil gebildeten dimeren Methacroleins gegenüber Verfahren des Standes der Technik vermindert wird, und dass die Bildung von Oligomeren oder Polymeren des Methacroleins fast vollständig vermieden wird.

Dies hat nicht nur eine große Bedeutung in Bezug auf die dadurch gesteigerte Ausbeute, die sich darin begründet, dass die Dimerisierung, Oligomerisierung oder Polymerisation des Produktes vermieden wird. Insbesondere die Reduktion des dimeren Methacroleins hat eine große Bedeutung in Bezug auf eventuelle Folgereaktionen. So wird Methacrolein, wie bereits in den Ausführungen zum Stand der Technik dargestellt, insbesondere in einer Oxidation zur Methacrylsäure weiterverarbeitet. Für die in diesen Oxidationsprozessen verwendeten Katalysatoren hat sich das dimere Methacrolein jedoch als Katalysatorgift erwiesen. Damit wiederum sinkt entweder die Standzeit der Katalysatoren oder aber es müssen zusätzliche weitere Reinigungsschritte zwischen den beiden Reaktionsstufen durchgeführt werden, die u.a. zusätzlich die Ausbeute senken.

Jedoch auch für eine alternative Verwendung des Methacroleins in einer oxidativen Veresterung z.B. zu Methylmethacrylat (MMA) ergeben sich zusätzliche Nachteile. So stellt man an einem so hergestellten MMA eine unerwünschte Gelbfärbung, insbesondere nach Lagerung fest. Eine solche Verfärbung kann mit einem auf Basis aus erfindungsgemäßen Methacrolein oder auf Basis eines anderen Methacroleins mit nur sehr geringem Dimeranteil hergestellten MMA nicht feststellen.

Weiterhin wurde überraschend gefunden, dass man insbesondere gegenüber Verfahren des Standes der Technik neben der höheren Reinheit und Ausbeute - eine deutliche Energieersparnis erzielen kann.

Ein großer Vorteil der vorliegenden Erfindung ist weiterhin, dass das Verfahren mit relativ einfachen und kostengünstigen Anlagekomponenten durchgeführt werden kann. Die Komponenten sind mit geringen Investitionskosten verbunden. Hierbei sind die Anlagen einfach zu warten und verursachen geringe Unterhaltskosten.

Das gewonnene Methacrolein kann nach der Entnahme aus dem Austritt des Rohrreaktors gereinigt werden. Insbesondere kann eine solche Reinigung mittels mindestens einer Destillation und mindestens einer, wie beschrieben zumeist an die erste Destillation anschließende Phasentrennung gereinigt werden. Anschließend kann das gereinigte Methacrolein insbesondere in einer Gasphasenoxidation zu Methacrylsäure umgesetzt werden. Alternativ und genauso bevorzugt wird das gereinigte Methacrolein in einer oxidativen Veresterung zu Methylmethacrylat umgesetzt.

### Beispiele

Das erfindungsgemäße Verfahren und die sich daraus ergebenden Vorteile werden im Folgenden anhand dieser Beispiele weiter beschrieben, ohne dass diese in irgendeiner Form einschränkend auf die Erfindung zu interpretieren sind.

### Beispiel 1 bis 9 / Vergleichsbeispiele 1 bis 11 (siehe Tabelle 1)

Eine Formalinlösung mit einem Formaldehydgehalt je nach Beispiel von 37 Gew% oder 55 Gew% und Propionaldehyd werden mittels eines statischen Mischers vermischt (im Weiteren als Aldehydlösung bezeichnet) und anschließend in einem ölbeheizten Wärmetauscher auf die gewünschte Temperatur (siehe Tab.1) erwärmt. Der genaue Wassergehalt des Formalins, abhängig vom Beispiel, spielt keine weitere Rolle, da dieser in den Wassergehalt des Frisch-Feeds gemäß Tabelle 1 vollständig mit eingeht. Ein Recyclestrom aus dem Sumpf der Produktkolonne, die an den Rohrreaktor anschließt, wird mit Essigsäure und Dimethylamin (als 40 %ige Lösung in Wasser) vermischt und ebenfalls auf die gewünschte Temperatur vorgewärmt. Die vorgewärmte Aldehydlösung und die vorgewärmte Katalysatorlösung werden in einem weiteren statischen Mischer vermischt. Diese Eduktmischung wird dann einem mittels Öl temperierten Rohreaktor zugeführt. Üblicherweise wird die Reaktion bei Drücken von ca. 35 bis 40 bar durchgeführt. Die Produktmischung am Ablauf des Rohrreaktors wird über ein Ventil entspannt und gelangt in die Produktkolonne zur Destillation. Am Kopf dieser Kolonne wird nach Kondensation und Phasentrennung ein zweiphasiges Gemisch aus Methacrolein und einer wässrigen Phase erhalten. Die wässrige Phase wird in die Kolonne zurückgeführt. Die organische Phase gelangt in die Produktvorlage. Am Sumpf der Kolonne wird ein Teilstrom als Recycle in die Reaktion zurückgeführt. Ein weiterer Teilstrom wird als wässriges Produkt in eine weitere Produktvorlage abgeführt.

In Beispiel 1 bis 4 wird entsprechend dem erfindungsgemäßen Verfahren ein Methacrolein mit einem DIMAL-Gehalt kleiner 0,2 Gew% erhalten. Der Wassergehalt beträgt ca. 56 Gew% und der auf das Wasser im Feed bezogene Dimethylamin-Gehalt beträgt ca. 2,7 Gew%. Die Temperatur im Reaktor liegt zwischen 122°C als Eingangstemperatur und 153°C als Austrittstemperatur. Eine erhebliche Temperaturspitze tritt nicht auf.

Beispiele 5 bis 7 sind weniger bevorzugt, da hier zwar ein Gehalt an dimeren MAL unter 0,4 Gew%, nicht jedoch unterhalb von 0,2 Gew% realisiert werden konnte. Der Unterschied zu den Beispielen 1 bis 4 ist hier neben einer teilweise höheren Eingangstemperatur vor allem die höheren Maximaltemperatur und Ausgangstemperatur.

Beispiele 8 und 9 sind noch weniger bevorzugt, da hier zwar ein Gehalt an dimeren MAL unter 0,5 Gew%, nicht jedoch unterhalb von 0,4 oder sogar 0,2 Gew% realisiert werden konnte. Hier waren die Eingangstemperaturen und insbesondere die Maximaltemperaturen noch höher. Insbesondere lagen die Maximaltemperaturen oberhalb der bevorzugten Maximaltemperaturen von 165 °C oder sogar von 170°C.

Vergleichsbeispiel 1 führt zwar zu einem Produkt mit 0,49 Gew%, das die dieser Erfindung zugrunde liegende Aufgabe, eine DIMAL-Gehalts kleiner 0,5 Gew% zu realisieren, noch löst, dabei jedoch aufgrund einer zu geringen Amin-Konzentration nicht erfinderisch ist. Wenn man diese Ergebnisse mit denen des sehr ähnlichen Beispiels 8, welches zur gleichen DIMAL-Konzentration im Produkt führt, vergleicht, so sind die Selektivität der Reaktion sowie der Umsatz schlechter. Weiterhin stellt man fest, dass in Vergleichsbeispiel 1 die Verweilzeiten kürzer und die Temperaturen geringer sind als bei der Durchführung des Beispiels 8. Daher sollte man eigentlich eine merklich geringere DIMAL-Konzentration erwarten. Da dem jedoch nicht so ist, geht hieraus klar hervor, dass auch die Amin-Konzentration überraschend eine große Rolle bei der Bildung des Nebenproduktes spielt.

Vergleichsbeispiel 4 wurde bei einer ebenfalls zu geringen Amin-Konzentration mit relativ hohen, wenn auch noch in den jeweils erfindungsgemäßen Grenzen befindlichen Temperaturen und Verweilzeiten durchgeführt. Damit war die Wärmebelastung der Reaktionsmischung gegenüber Vergleichsbeispiel 1 deutlich höher und es resultierte sogar eine DIMAL-Gehalt von größer 0,9 Gew%.

Vergleichsbeispiel 2 und 3 wiesen relativ geringe DMA-Gehalte auf. Insbesondere wurden diese aber mit einer Eingangstemperatur deutlich über 150 °C durchgeführt. Entsprechend resultierte ein DIMAL-Gehalt von sogar deutlich größer 1,0 Gew%. Vergleichsbeispiele 4 und 7 wurden mit einem zu geringen Gehalt an organischer Base (DMA) durchgeführt. Die Vergleichsbeispiele 5 bis 7 wurden dagegen bzw. zusätzlich mit einem Wassergehalt, der nicht mehr erfindungsgemäß unterhalb von 50 Gew% lag, durchgeführt. All die Beispiele führten zu Produkten mit vergleichbaren Selektivitäten und Umsätzen bei gleichzeitig sehr hohen DIMAL-Gehalten im Produkt von mehr als 0,6 Gew%.

Vergleichsbeispiele 8 und 9 wurden wiederum nicht erfindungsgemäß mit Eingangstemperaturen oberhalb von 150 °C und entsprechend sehr hohen Maximal- und Ausgangstemperaturen durchgeführt. Die DIMAL-Gehalte waren daraufhin zu hoch, obwohl mit einer sehr verdünnten Fahrweise, kurzen Verweilzeiten und einer relativ hohen Amin-Konzentration den hohen Eingangstemperaturen entgegengesteuert wurde.

### Vergleichsbeispiel 10 (Batchverfahren)

In einem 1 L Autoklaven werden Propionaldehyd und Formaldehyd, in Form von Formalin (in einem molaren Verhältnis von 1:1) vorgelegt. Der mittels Ölbad temperierte Autoklav wird verschlossen und mit 40 bar Stickstoff beaufschlagt. Unter Rühren wird der Inhalt auf ca. 120 °C aufgeheizt. Bei Erreichen der Solltemperatur wird die Katalysatorlösung aus Wasser, Dimethylamin und Essigsäure (0,07 Teile Dimethylamin auf ein Teil Propionaldehyd , sowie ein Verhältnis Säure zu Base von 1,1 zu 1,0) hinzugegeben. Im Feed betrug die Wasserkonzentration ca. 56 Gew%, die Beladung des Wassers mit Dimethylamin betrug 2,5 Gew%. Nach ca. 20 min wird der Versuch abgebrochen und der Autoklav in einem gerührten Eisbad gekühlt. Das Gemisch wird entnommen und mittels Phasentrennung in eine organische und eine wässrige Phase getrennt. Beide Phasen werden hinsichtlich Ihrer Zusammensetzung untersucht. Der Propionaldehyd-Umsatz beträgt 99,8 Gew%, die MAL-Ausbeute beträgt 75,9 Gew% und der DIMAL-Gehalt des Methacroleins beträgt 11,26 Gew%.

### Vergleichsbeispiele 11, 12 und 13 (Batchversuche; siehe Tabelle 2)

In einem 0,45 L Autoklaven werden Propionaldehyd und Formaldehyd in Form von Formalin (molares Verhältnis von 1:1) vorgelegt. Der mittels Ölbad temperierte Autoklav wird verschlossen und mit 40 bar Stickstoff beaufschlagt. Unter Rühren wird der Inhalt auf ca. 115 °C aufgeheizt. Bei Erreichen der Solltemperatur wird die Katalysatorlösung aus Wasser, Dimethylamin und Essigsäure hinzugegeben. Nach einer gewünschten Zeit wird der Versuch abgebrochen und der Autoklav in einem gerührten Eisbad gekühlt. Das Gemisch wird entnommen und mittels Phasentrennung in eine organische und eine wässrige Phase getrennt. Beide Phasen werden hinsichtlich Ihrer Zusammensetzung untersucht. Die Ergebnisse sind in Tabelle 2 zusammengefasst.

**Tabelle 2: Batch-Experimente (Vergleichsbeispiele 11 bis 13)**

| | PA:FO | DMA:PA | ACOH:DMA | H2O | DMA/H2O | VWZ | TÖL | X PA | SMAL | c DIMAL |
|---|---|---|---|---|---|---|---|---|---|---|
| | mol/mol | mol% | mol/mol | Gew% | Gew% | min | °C | Gew% | Gew% | Gew% |
| VB 11 | 1 | 0,065 | 1,10 | 68 | 1,4 | 34 | 115 | 99,4 | 86 | 4,8 |
| VB 12 | 1 | 0,075 | 1,10 | 68 | 1,5 | 16 | 115 | 98,8 | 90 | 2,9 |
| VB 13 | 1 | 0,075 | 1,10 | 68 | 1,5 | 2 | 115 | 98,3 | 87 | 1,7 |

Gemäß den in Tabelle 2 dargestellten Ergebnissen ist die Durchführung der Reaktion bei einer Gesamttemperatur von 115 °C nicht interessant, da in allen drei Vergleichsbespielen 11 bis 13 die gewünschte DIMAL-Konzentration im Produkt zu hoch ist. Weiterhin kommt man in einem Batchverfahren aufgrund der nötigen längeren Verweilzeit zu schlechteren Ausbeuten und/oder zu höheren Konzentrationen an dimeren MAL:
Die Beispiele zeigen insbesondere zu den Ergebnissen der Vergleichsbeispiele, dass insbesondere die Kombination der Merkmale des ersten Anspruchs zu sehr guten Ausbeuten mit gleichzeitig geringem Anteil an dimeren Methacrolein im Produkt führen.

| **Tabelle 1** | PA:FO | DMA:PA | ACOH:DMA | Recycle | DMA:PA | H₂O | DMA/H2O | VWZ | T_{ÖL} | Tₑᵢₙ | Tₘₐₓ | Taus | Umsatz PA | Selkt. MAL | c DIMAL |
|---|---|---|---|---|---|---|---|---|---|---|---|---|---|---|---|
| | Frischfeed | | | | Reaktoreingang | | | | | | | | | | |
| | mol/mol | mol% | mol/mol | % | mol% | % | % | sec | °C | °C | | °C | % | % | % |
| DE3213681A1 Bsp1 | 1 | 3,7 | 1,08 | - | - | 50 | 1,8 | 6,9 | | 161 | 184 | - | 99,5 | 98,1 | 0,49 |
| DE3213681A1 Bsp2 | 1 | 3,6 | 1,14 | - | - | 40 | 2,5 | 6 | | 162 | 205 | - | > 99,4 | 97,2 | <1 |
| Beispiel 1 | 0,99 | 2,50 | 1,09 | 70,5 | 7,8 | 55,6 | 2,74 | 9,30 | 139,5 | 122,5 | 152,6 | 152,2 | 99,37 | 98,75 | 0,18 |
| Beispiel 2 | 0,99 | 2,51 | 1,09 | 71,0 | 7,8 | 56,1 | 2,74 | 9,26 | 139,1 | 122,5 | 152,3 | 152,0 | 99,30 | 98,85 | 0,18 |
| Beispiel 3 | 0,98 | 2,61 | 1,09 | 71,2 | 8,2 | 54,9 | 2,82 | 9,41 | 139,9 | 122,1 | 152,3 | 152,2 | 99,35 | 98,67 | 0,18 |
| Beispiel 4 | 0,96 | 2,51 | 1,09 | 70,1 | 7,7 | 56,5 | 2,71 | 9,21 | 139,1 | 122,8 | 153,0 | 153,0 | 99,46 | 98,33 | 0,18 |
| Beispiel 5 | 0,99 | 2,51 | 1,09 | 70,5 | 7,8 | 55,7 | 2,75 | 9,26 | 143,9 | 129,9 | 160,2 | 155,5 | 99,75 | 98,19 | 0,34 |
| Beispiel 6 | 0,99 | 2,51 | 1,09 | 70,4 | 7,8 | 55,6 | 2,75 | 9,30 | 144,2 | 127,3 | 157,7 | 154,7 | 99,65 | 98,47 | 0,27 |
| Beispiel 7 | 0,98 | 2,50 | 1,09 | 70,5 | 7,7 | 56,0 | 2,72 | 9,22 | 139,0 | 122,5 | 156,3 | 154,9 | 99,57 | 98,62 | 0,22 |
| Beispiel 8 | 0,99 | 2,51 | 1,09 | 70,5 | 7,8 | 55,6 | 2,74 | 9,26 | 159,8 | 142,1 | 173,0 | 169,1 | 99,67 | 98,03 | 0,49 |
| Beispiel 9 | 0,99 | 2,52 | 1,08 | 70,4 | 7,8 | 55,6 | 2,76 | 9,26 | 146,4 | 133,8 | 165,4 | 159,7 | 99,77 | 98,34 | 0,45 |
| VB 1 | 0,99 | 2,50 | 1,09 | 48,8 | 4,7 | 56,4 | 1,76 | 9,23 | 159,5 | 141,0 | 172,3 | 169,6 | 99,65 | 98,02 | 0,49 |
| VB 2 | 1,01 | 2,49 | 1,10 | 52,8 | 5,2 | 58,4 | 1,73 | 11,26 | 164,1 | 155,7 | 179,6 | 172,7 | 99,81 | 98,51 | 1,35 |
| VB 3 | 1,00 | 2,52 | 1,09 | 53,5 | 5,2 | 58,7 | 1,73 | 11,23 | 164,0 | 150,3 | 177,5 | 168,7 | 99,83 | 98,40 | 1,13 |
| VB 4 | 1,00 | 2,50 | 1,11 | 49,3 | 4,8 | 68,5 | 1,75 | 9,33 | 158,2 | 147,2 | 173,0 | 164,4 | 99,81 | 97,91 | 0,92 |
| VB 5 | 1,00 | 4,83 | 1,10 | 16,5 | 5,7 | 41,8 | 3,78 | 12,27 | 149,1 | 150,5 | 179,8 | 167,8 | 99,32 | 96,44 | 0,93 |
| VB 6 | 0,99 | 4,81 | 1,09 | 16,7 | 5,7 | 42,3 | 3,73 | 12,21 | 148,4 | 142,2 | 172,2 | 158,6 | 99,17 | 98,05 | 0,65 |
| VB 7 | 0,99 | 4,01 | 1,10 | 16,6 | 4,8 | 41,7 | 3,11 | 12,32 | 153,6 | 141,5 | 171,9 | 159,2 | 99,66 | 98,58 | 0,68 |
| VB 8 | 1,00 | 2,50 | 1,10 | 65,3 | 6,7 | 83,9 | 1,66 | 7,66 | 158,8 | 154,2 | 171,7 | 171,7 | 99,58 | 98,18 | 0,94 |
| VB 9 | 1,01 | 2,50 | 1,10 | 65,0 | 6,5 | 64,8 | 1,63 | 7,68 | 158,9 | 150,3 | 171,7 | 171,7 | 99,68 | 98,34 | 0,65 |

## Patentansprüche

1. Verfahren zur kontinuierlichen Durchführung einer Mannich-Reaktion, bei der aus Formaldehyd und Propionaldehyd mit mindestens einer Säure und mindestens einem Amin als Katalysatoren Methacrolein hergestellt wird, **dadurch gekennzeichnet, dass**
die Reaktion in einem kontinuierlich betriebenen Rohrreaktor oder Plattenreaktor mit Temperaturgradienten durchgeführt wird, wobei die Eingangstemperatur des Zulaufs der Reaktanten und Katalysatoren in den Reaktor zwischen 100 und kleiner 150 °C und die Temperatur der Reaktionsmischung im Ablauf des Reaktors maximal 180 °C beträgt,
der Wassergehalt im Zulauf des Reaktors größer 45 Gew% und maximal 85 Gew% ist,
die Menge an organischer Base im Zulauf des Reaktors, umfassend die reine Base, sowie den jeweiligen Basenanteil in den Salzen mit der Säure und in den basenhaltigen Zwischenprodukten der Mannich-Reaktion, bezogen auf Propionaldehyd mehr als 5 Mol% beträgt,
der Innendruck des Reaktors so eingestellt ist, dass dieser größer ist als der Siededruck der Reaktionsmischung
und die Verweilzeit der Reaktionsmischung in dem Reaktor zwischen 1 und 30 s liegt.

2. Verfahren gemäß Anspruch 1, **dadurch gekennzeichnet, dass** der Zulauf ein Verhältnis von Propionaldehyd zu Formaldehyd zwischen 0,75 zu 1 mol und 1 zu 0,8 mol aufweist.

3. Verfahren gemäß Anspruch 1 oder 2, **dadurch gekennzeichnet, dass** mindestens 20 % der Gesamtmenge organischer Base des Zulaufs aus dem Frisch-Feed stammen, und dass der Zulauf bezogen auf ein mol organische Base zwischen 1 und 1,5 mol Säure enthält.

4. Verfahren gemäß Anspruch 3, **dadurch gekennzeichnet, dass** es sich bei der organischen Base um ein sekundäres Amin, bevorzugt um Dimethylamin und bei der Säure um Ameisensäure, Essigsäure und/oder Propionsäure handelt.

5. Verfahren gemäß mindestens einem der Ansprüche 1 bis 4, **dadurch gekennzeichnet, dass** die Maximaltemperatur im Reaktor maximal 170 °C beträgt.

6. Verfahren gemäß mindestens einem der Ansprüche 1 bis 5, **dadurch gekennzeichnet, dass** die Eingangstemperatur in den Reaktor zwischen 110 und 140 °C und die Temperatur der Reaktionsmischung im Ablauf zwischen 155 und 170 °C liegen.

7. Verfahren gemäß mindestens einem der Ansprüche 1 bis 6, **dadurch gekennzeichnet, dass** es sich bei dem Reaktor um Rohrreaktoren in Form eines Rohrbündelreaktors handelt, und dass das einzelne Rohr einen Innendurchmesser zwischen 4 und 20 mm aufweist.

8. Verfahren gemäß mindestens einem der Ansprüche 1 bis 7, **dadurch gekennzeichnet, dass** der Rohrreaktor mit eine Leerrohrgeschwindigkeit zwischen 0,3 und 2,0 m/s durchströmt wird und optional statische Mischer aufweist.

9. Verfahren gemäß mindestens einem der Ansprüche 1 bis 8, **dadurch gekennzeichnet, dass** die Methacroleinkonzentration der Reaktionsmischung am Ablauf zwischen 20 und 50 Gew% liegt.

10. Verfahren gemäß mindestens einem der Ansprüche 1 bis 9, **dadurch gekennzeichnet, dass** die umgesetzte Reaktionslösung nach der Entnahme am Austritt in einer Kolonne destilliert wird und anschließend das Methacrolein in einem Phasentrenngefäß von der sich abscheidenden wässrigen Phase getrennt wird, wobei diese wässrige Phase ganz oder teilweise zurück in die Kolonne geführt wird.

11. Verfahren gemäß Anspruch 10, **dadurch gekennzeichnet, dass** mindestens 20 Gew% der wässrigen Phase aus dem Sumpf der Kolonne in den Eintritt des Rohrreaktors recycliert werden

12. Verfahren gemäß mindestens einem der Ansprüche 1 bis 11, **dadurch gekennzeichnet, dass** die Verweilzeit der Reaktionsmischung in dem Reaktor zwischen 5 und 15 s liegt.

13. Verfahren gemäß mindestens einem der Ansprüche 1 bis 12, **dadurch gekennzeichnet, dass** das Methacrolein mittels mindestens einer Destillation und mindestens einer Phasentrennung gereinigt wird und anschließend in einer Gasphasenoxidation zu Methacrylsäure umgesetzt wird.

14. Verfahren gemäß mindestens einem der Ansprüche 1 bis 12, **dadurch gekennzeichnet, dass** das Methacrolein mittels mindestens einer Destillation und mindestens einer Phasentrennung gereinigt wird und anschließend in einer oxidativen Veresterung zu Methylmethacrylat umgesetzt wird.
